# FASCICULE DE BREVET EUROPEEN

(11) **EP 3 770 151 B1**
(45) Date de publication et mention de la délivrance du brevet: **18.05.2022**
(21) Numéro de dépôt: 20186796.7
(22) Date de dépôt: 20.07.2020
(51) Int. Cl.: C07D 311/04, C07D 413/04

(54) **PROCÉDÉ DE FABRICATION DE CHROMENES PAR THERMOLYSE DESTINÉS A LA PRÉPARATION DE RESINES THERMODURCISSABLES**
VERFAHREN ZUR HERSTELLUNG VON CHROMENEN DURCH THERMOLYSE, DIE FÜR DIE HERSTELLUNG VON WÄRMEHÄRTBAREN HARZEN BESTIMMT SIND
METHOD FOR MANUFACTURING CHROMENES BY THERMOLYSIS INTENDED FOR THE PREPARATION OF HEAT-SETTING RESINS

(30) Priorité: 23.07.2019 FR 1908324
(43) Date de publication de la demande: 27.01.2021
(73) Titulaire: ArianeGroup SAS, 78130 Les Mureaux (FR); CENTRE NATIONAL DE LA RECHERCHE SCIENTIFIQUE (C.N.R.S.), 75016 Paris (FR); Université De Reims Champagne-Ardenne, 51100 Reims (FR)
(72) Inventeur: POUYET, Robin, 63800 COURNON (FR); COQUERET, Xavier, 51100 REIMS (FR); DEFOORT, Brigitte, 33160 Saint-Médard-en-Jalles (FR); RIVIERES, Bastien, 81640 LE SEGUR (FR)
(74) Mandataire: Cabinet Beau de Loménie

(56) Documents cités:
- WO-A1-2017/129661
- WO-A2-01/10861
- AL-SADER ET AL.: "On the Mechanism of flash vacuum pyrolysis of phenyl propargyl ether. Intramolecular Deuterium kinetic isotope effect on Claisen Rearrangement", J. ORG. CHEM., vol. 43, no. 18, 1978, pages 3626-3627, XP055701628,
- KATHLYN A. PARKER ET AL: "Electrocyclic Ring Closure of the Enols of Vinyl Quinones. A 2H -Chromene Synthesis", ORGANIC LETTERS, vol. 3, no. 24, 1 novembre 2001 (2001-11-01), pages 3875-3878, XP055701593, ISSN: 1523-7060, DOI: 10.1021/ol0167199

## Description

La présente invention concerne le domaine des résines thermodurcissables, et des matériaux obtenus à partir de ces résines. Ces résines visent à remplacer les résines phénoliques dans toutes les applications dans lesquelles celles-ci sont utilisées, et notamment les matériaux dits « ablatifs ».

Un matériau ablatif est défini comme un matériau qui est capable de subir une ablation, c'est-à-dire une perte de substance par décomposition chimique, changement d'état ou érosion mécanique sous l'effet d'un flux de matière ou de rayonnement (Journal Officiel de la République Française du 22 septembre 2000). C'est en particulier le cas des matériaux qui entrent dans la constitution des boucliers thermiques destinés à l'aérospatiale et des parois de tuyères des moteurs à propulsions. Typiquement, dans ce cas, la couche externe du matériau ablatif qui est directement en contact avec l'environnement, subit sous l'effet de la chaleur une transformation chimique, ainsi qu'une récession liée à cette transformation. Cette couche externe rayonne donc vers l'extérieur et sa transformation chimique consomme de l'énergie. Ces deux effets contribuent à une moindre transmission de la chaleur vers les couches internes du matériau et donc à une isolation thermique de la structure sous-jacente. Un bon matériau ablatif doit être tel que sa transformation chimique sous l'effet de la chaleur soit endothermique, sa conductivité thermique soit faible en régime stationnaire et/ou en transitoire et sa transformation chimique ne s'accompagne pas d'une récession trop rapide. En particulier, pour remplir ce dernier point, il faut que la transformation chimique du matériau ablatif s'accompagne de la formation d'une croûte à base de carbone ou silice provenant de la pyrolyse de la résine.

Ceci est en particulier obtenu pour des résines ayant un taux de coke élevé. Le taux de coke est défini comme la masse de résidu qui est obtenu quand on décompose un échantillon d'un polymère organique par pyrolyse, à une température de 900°C sous atmosphère inerte (azote ou argon), rapportée à la masse initiale de cet échantillon. Les résines les plus intéressantes présentent un taux de coke supérieure à 50%.

Les résines phénoliques présentent en général un tel taux de coke et sont obtenues par polycondensation de monomères issus de la pétrochimie : le phénol et le formaldéhyde, ce qui leur vaut d'être aussi appelées résines phénol-formaldéhydes ou résines formophénoliques. Les précurseurs des résines phénoliques, le phénol et le formaldéhyde, sont respectivement CMR 2 et 1B. Ces deux composés sont donc sous la surveillance du Règlement (CE) n° 1907/2006 du Parlement Européen (REACH) qui vise à mieux protéger la santé humaine et l'environnement contre les risques liés aux substances chimiques. Il s'avère, de plus, que la polycondensation du phénol et du formaldéhyde n'est jamais achevée, d'où la présence de composés volatils et de molécules d'eau qui sont très difficiles à éliminer si un cycle thermique bien défini n'est pas suivi au cours de cette polycondensation et qui peuvent conduire à des matériaux poreux dans leur état natif ainsi qu'à des dégazages durant la vie des matériaux fabriqués à partir de résines phénoliques. Or, ces dégazages peuvent avoir des conséquences très néfastes dans certaines applications comme, par exemple, les applications aérospatiales.

Compte-tenu de la place qu'occupent actuellement les résines phénoliques dans l'industrie des matériaux plastiques et de ses inconvénients, de nouvelles résines thermodurcissables présentant des propriétés similaires à celles des résines phénoliques ont été obtenues à partir de précurseurs différents. Ainsi la demande de brevet WO2017/129661 décrit de telles résines et leurs procédés de fabrication. De telles résines ont un taux de coke supérieure à 50% et peuvent donc être utilisées comme matériaux ablatifs. Les précurseurs utilisés sont en particulier des molécules aromatiques porteuses de fonctions éther de propargyle. Toutefois l'énergie trop importante libérée pendant leur polymérisation pourrait engendrer un emballement thermique lors de la fabrication des matériaux composites. Ainsi, afin d'obtenir une enthalpie de polymérisation d'environ 800-900 J/g avec une perte de masse la plus faible possible lors de la polymérisation, il est nécessaire dans le procédé décrit dans cette demande de maintenir un traitement thermique long pendant la polymérisation afin de prévenir tout emballement thermique. Cette solution n'est donc pas optimisée vis-à-vis de la fabrication des pièces épaisses pouvant mesurer jusqu'à plusieurs dizaines de millimètres d'épaisseurs.

Les inventeurs se sont rendus compte qu'il était possible de diminuer l'énergie libérée pendant la polymérisation des résines à terminaisons éther de propargyle d'un facteur 6 par la conversion de la fonction éther de propargyle en fonction chromène et ainsi d'abaisser l'enthalpie de polymérisation jusqu'à une valeur < 500 J/g.

Les inventeurs se sont aperçus de façon surprenante qu'il était possible de faire une telle conversion à l'aide d'un procédé innovant de thermolyse. L'objectif de cette voie de synthèse est de s'affranchir de l'utilisation de catalyseur potentiellement toxique et onéreux, par chauffage intense et bref des fonctions éther de propargyle.

Des procédés de thermolyse ont déjà été décrits dans l'art antérieur à partir de composés ayant des fonctions éthers de propargyle, mais sans arriver à obtenir des chromènes (Al-Sader et al., J. Org. Chem., 1978, Vol.43, N°18, Communications, pages 3626-3627 et Trahanovsky et al., 1968, Journal of the American Chemical Society, 90(11), 2839-2842).

La demande de brevet WO01/10861 décrit un procédé de préparation de chromènes par pyrolyse des éthers de propargyle aromatique correspondants à une température comprise entre 150 et 250°C.

L'article de Kathlyn et al. (ORGANIC LETTERS, vol.3 n°24, 1 novembre 2001 pages 3875-3878) décrit l'obtention de chromènes par thermolyse de vinyl quinones.

Les inventeurs se sont aperçus qu'il était possible d'obtenir des chromènes lors de l'utilisation d'un tel procédé en utilisant des produits de départ particuliers.

La présente invention concerne donc un procédé de fabrication de chromènes destinés à la préparation de résines thermodurcissables comprenant l'étape de transformation d'un éther de propargyle aromatique de formule générale (I) suivante 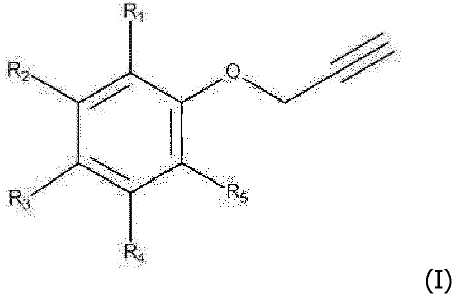 dans laquelle :
**R₁ et R₅** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₁ et R₅** représente un atome d'hydrogène ;
**R₂ et R₄** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle ;
**et R₃** représente un atome d'hydrogène, un groupe O-alkyle en C₁-C₆ ou un groupe alcène en C₂-C₆, le groupe alcène étant éventuellement substitué par un groupe de formule générale (II) suivante 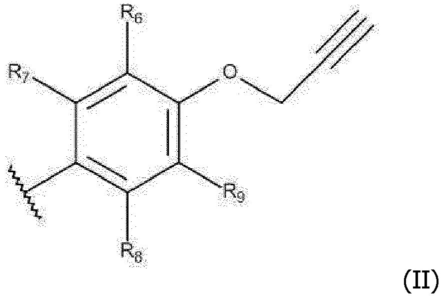 dans laquelle :
   **R₆ et R₉** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₆ et R₉** représente un atome d'hydrogène ;
   et **R₇ et R₈** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle;
   à la condition que l'un au moins des **R₁, R₂, R₃, R₄ et R₅** ne représente pas un atome d'hydrogène ou un groupe O-alkyle en C₁-C₆ ;
   et ses isomères cis/trans et ses isomères optiques et leurs mélanges racémiques
   en un chromène par thermolyse éclair sous vide, à une température comprise entre 300 et 600°C, avantageusement entre 400 et 450°C, sous une pression comprise entre 3 et 10000 Pa, avantageusement entre 4 et 10 000 Pa, plus avantageusement entre 5 et 7000 Pa.

Au sens de la présente invention on entend par « groupe alcène en C₂-C₆ » tout groupe alcène de 2 à 6 atomes de carbones, linéaire ou ramifié, en particulier le groupe vinyle, le groupe allyle ou le groupe but-2-ényle. Au sens de la présente invention on entend par « groupe alcyne en C₂-C₆ » tout groupe alcyne de 2 à 6 atomes de carbones, linéaire ou ramifié, en particulier le groupe éthynyle ou le groupe propargyle.

Au sens de la présente invention on entend par « groupe O-alkyle en C₁-C₆ » tout groupe O-alkyle de 1 à 6 atomes de carbones, linéaire ou ramifié, en particulier, le groupe méthoxy ou éthoxy.

Au sens de la présente invention on entend par « groupe O-alcène en C₂-C₆ » tout groupe O-alcène de 2 à 6 atomes de carbones, linéaire ou ramifié.

Au sens de la présente invention on entend par « groupe O-alcyne en C₂-C₆ » tout groupe O-alcyne de 2 à 6 atomes de carbones, linéaire ou ramifié en particulier le groupe O-propargyle.

Avantageusement l'éther de propargyle aromatique de formule générale (I) est choisi dans le groupe constitué par le résorcinol propargylé, l'eugénol propargylé, l'eugénol couplé propargylé, l'isoeugénol couplé propargylé, l'isoeugénol propargylé et leurs mélanges et leurs isomères cis/trans et leurs isomères optiques et leurs mélanges racémiques, plus avantageusement il s'agit du résorcinol propargylé. Ces produits sont bien connus de l'homme du métier et peuvent être préparés par des procédés bien connus, tels que ceux décrits dans la demande WO2017/129661. Ils ont l'avantage de pouvoir être issus de composés biosourçables tels que le résorcinol, l'eugénol, l'eugénol couplé, l'isoeugénol et l'isoeugénol couplé.

Le résorcinol propargylé a ainsi la formule générale suivante :

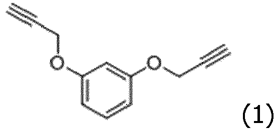

L'eugénol propargylé a ainsi la formule générale suivante :

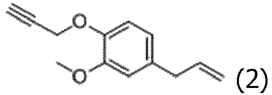

L'eugénol couplé propargylé a ainsi la formule générale suivante : 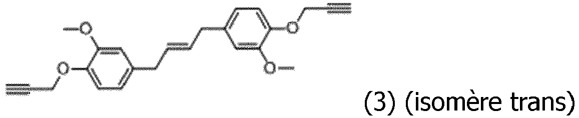 ou la formule générale suivante 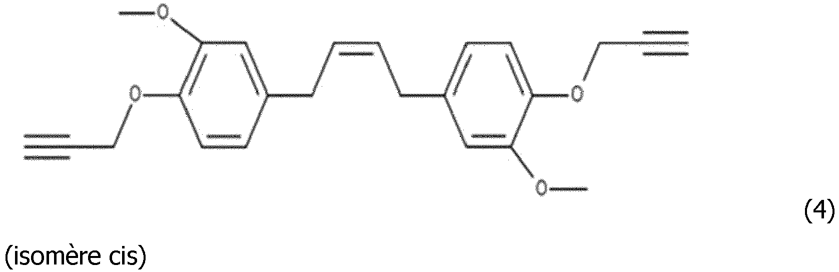 ou un mélange de ces deux isomères.

L'isoeugénol propargylé a ainsi la formule générale suivante :

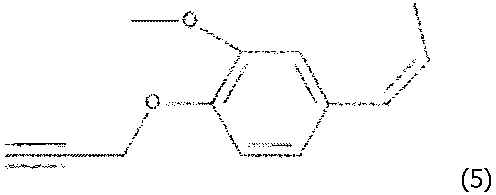

L'isoeugénol couplé propargylé a ainsi la formule générale suivante : 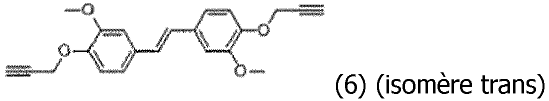 ou la formule générale suivante 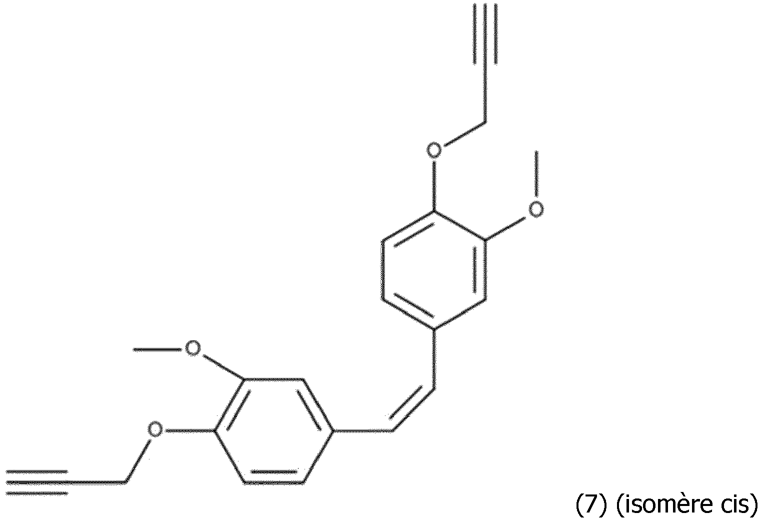 ou un mélange de ces deux isomères.

La thermolyse peut avoir lieu en présence d'un solvant. Ce solvant doit pouvoir solubiliser l'éther de propargyle aromatique de formule générale (I) qui se trouve donc sous forme dissoute lors de la thermolyse. Ce solvant doit également être inerte aux températures mises en jeu. Il sert de gaz dispersant. Ce solvant peut avantageusement être un solvant organique tel que le toluène. Dans un mode de réalisation avantageux, l'éther de propargyle aromatique de formule générale (I) est au préalable solubilisé dans un solvant, avantageusement du toluène, avant l'étape de thermolyse éclair. Avantageusement, le procédé selon la présente invention comprend une étape supplémentaire d'élimination du solvant, en particulier par évaporation.

La thermolyse peut également avoir lieu sans utilisation de solvant. Dans ce cas l'éther de propargyle aromatique de formule générale (I) peut être introduit dans le dispositif de thermolyse par gravité ou par évaporation/sublimation, avantageusement par évaporation/sublimation. Dans un mode de réalisation avantageux, l'éther de propargyle aromatique de formule générale (I) est au préalable évaporé/sublimé avant l'étape de thermolyse éclair, par exemple à une température de 130°C et une pression de 5,5 Pa lorsque le composé de formule générale (I) est du résorcinol propargylé.

La température de la thermolyse éclair est comprise entre 300 et 600°C, avantageusement entre 350 et 550°C, plus avantageusement entre 380 et 500°C, en particulier entre 400 et 450°C.

La pression de la thermolyse éclair est comprise entre 3 et 10000 Pa, avantageusement entre 4 et 10 000 Pa, plus avantageusement entre 5 et 7000 Pa. Dans le cas où la thermolyse éclair est mise en œuvre en présence d'un solvant, la pression est avantageusement comprise entre 4000 et 10 000 Pa, plus avantageusement entre 5000 et 7000 Pa, encore plus avantageusement elle est de 6000 Pa. Dans le cas où la thermolyse éclaire est mise en œuvre sans solvant et où le produit est au préalable évaporé/sublimé, la pression est avantageusement comprise entre 4 et 10 Pa, plus avantageusement entre 4,5 et 7 Pa, encore plus avantageusement elle est de 5,5 Pa.

Le principe du procédé de thermolyse éclair selon l'invention est d'éloigner les molécules spatialement pour favoriser les réactions intramoléculaires, et supprimer les réactions intermoléculaires parasites, les réactions intermoléculaires comprennent notamment les réactions de polymérisation entre motifs chromène. Cet éloignement spatial se fait par une mise sous vide de la colonne de réaction, le réactif se retrouvant en phase gaz lors de son réarrangement intramoléculaire. Les temps de réaction sont très faibles, généralement inférieur à la seconde.

Le procédé selon l'invention consiste donc à introduire un précurseur en phase gazeuse dans une colonne préchauffée à très haute température puis de condenser rapidement le produit en sortie de colonne. La colonne de réaction est généralement remplie d'un solide inerte, comme des billes de quartz ou de verres, afin d'assurer l'échange thermique entre le réactif et le four. Avantageusement, la colonne est une colonne en verre de longueur et de largeur définies, par exemple de longueur entre 55 et 75 cm, préférentiellement entre 60 et 65 cm, en particulier de 64 cm et de diamètre compris entre 20 et 40mm, en particulier de 30mm, remplie par exemple de billes de verre de diamètre compris entre 2 et 10 mm, préférentiellement 4mm (±0,3mm).

Le dispositif peut en outre comprendre un four tubulaire vertical, un récipient d'accueil de volume suffisant refroidit par azote liquide. Il peut de plus comprendre une ampoule de coulée par laquelle introduire le réactif lorsque la thermolyse est mise en œuvre en présence de solvant, et une connexion à une pompe à membrane, permettant d'atteindre le vide nécessaire. Il peut par ailleurs comprendre à la place de la pompe à membrane une pompe à palette pour obtenir un vide plus poussé et un moyen de chauffe du réactif (tel qu'une bande chauffante, un four ou un pistolet thermique) dans le cas où ce dernier est évaporé/sublimé avant la thermolyse ainsi qu'un tube horizontal d'introduction du réactif. L'appareillage utilisé pour la thermolyse éclair est bien connu de l'homme du métier et est en particulier décrit dans l'art antérieur.

Les inventeurs se sont aperçus qu'il n'était pas nécessaire d'avoir une conversion quantitative des fonctions éthers de propargyle en chromène pour obtenir une enthalpie de polymérisation inférieure à 500J/g. En effet l'énergie libérée pendant la polymérisation pour un substrat donné est dépendant de sa masse molaire et de sa fonctionnalité. En utilisant l'énergie libérée par fonction propargyle et par fonction chromène, combinée à la masse molaire de chaque substrat, il est possible de déterminer théoriquement le pourcentage de fonctions propargyle résiduelles maximum afin de se trouver en dessous des 500 J/g. ces valeurs ont été comparées aux valeurs expérimentales obtenues et sont similaires. Ainsi le tableau 1 ci-après indique le pourcentage de fonctions propargyle résiduelles théorique afin de se trouver en dessous des 500 J/g d'enthalpie de réaction lors de la polymérisation. Le pourcentage de fonctions propargyles résiduelles théorique est calculé de la façon suivante : (nombre de moles de fonctions propargyles à l'issue de la réaction) / (nombre de moles de fonctions propargyle avant le début de la réaction) ×100.

**Tableau 1**

| Substrat | M (g/mol) | Fonctionnalité en groupements propargyle | Pourcentage molaire de fonctions propargyles résiduelles théorique pour atteindre une enthalpie de 500 J/g |
|---|---|---|---|
| Résorcinol propargylé | 186,21 | 2 | 11 |
| Eugénol couplé propargylé | 376,44 | 2 | 39 |
| Isoeugénol couplé propargylé | 348,39 | 2 | 35 |

Ainsi avantageusement, la conversion des éthers de propargyles aromatiques en chromène par le procédé selon la présente invention n'est pas totale et le chromène obtenu comprend des fonctions propargyles résiduelles. De façon avantageuse, le pourcentage molaire de fonctions propargyles résiduelles du chromène est inférieur à 11% lorsque l'éther de propargyle aromatique de formule générale (I) est le résorcinol propargylé, le pourcentage molaire de fonctions propargyles résiduelles du chromène est inférieur à 39% lorsque l'éther de propargyle aromatique de formule générale (I) est l'eugénol couplé propargylé et le pourcentage molaire de fonctions propargyles résiduelles du chromène est inférieur à 35% lorsque l'éther de propargyle aromatique de formule générale (I) est l'isoeugénol couplé propargylé.

Dans le cas où l'éther de propargyle aromatique de formule générale (I) est le résorcinol propargylé, le chromène obtenu à l'aide du procédé selon l'invention peut avoir la formule C et / ou D suivante, avantageusement il s'agit d'un mélange des formules C et D.

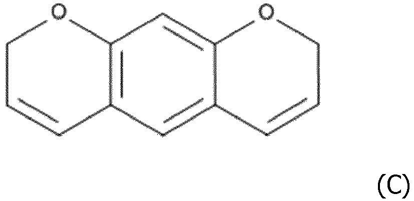

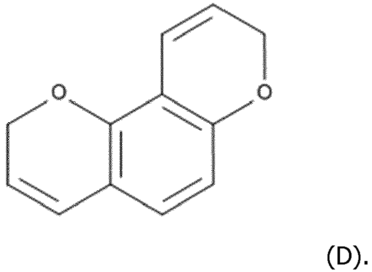

En particulier le procédé selon l'invention favorise la formation du composé D par rapport au composé C. Les proportions molaires peuvent ainsi être avantageusement comprises entre 75 et 96% en fonction des conditions opératoires.

Dans le cas où l'éther de propargyle aromatique de formule générale (I) est le résorcinol propargylé, le chromène obtenu à l'aide du procédé selon l'invention peut également avoir la formule A et / ou B suivante

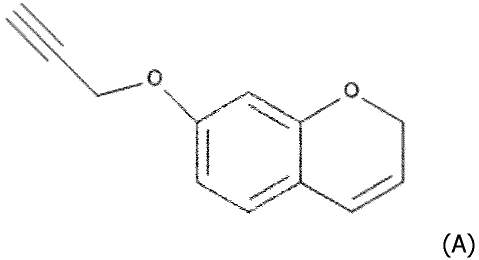

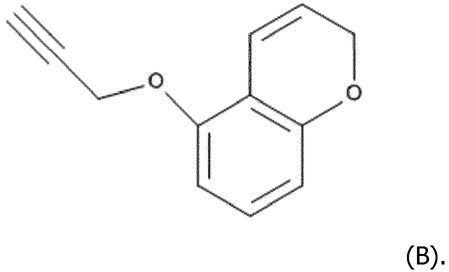

Toutefois ces molécules sont en général rapidement converties en composés de formule C et D.

Avantageusement, le rendement molaire de la réaction de conversion de l'éther de propargyle aromatique en chromène est compris entre 60 et 99%, en particulier entre 65 et 99%.

La présente invention concerne en outre un procédé de préparation d'un matériau en résine thermodurcie comprenant les étapes successives suivantes :
- a) mise en œuvre du procédé selon la présente invention, avantageusement tel que décrit ci-dessus ;
- b) polymérisation du produit de la réaction obtenu à l'étape a) de façon à obtenir le matériau en résine thermodurcie.
- c) récupération du matériau en résine thermodurcie obtenu à l'étape b).

Conformément à l'invention, la polymérisation de la résine peut être réalisée par tout moyen susceptible d'induire la polymérisation/réticulation du chromène et, notamment, par application d'un traitement thermique, ou d'un traitement lumineux (lumière visible, UV ou IR).

En particulier l'étape b) est mise en œuvre par traitement thermique, avantageusement à une température comprise entre 80°C et 180°C, plus avantageusement à l'aide de plusieurs paliers de chauffe (en particulier 5), sans ajout d'autres composants, tel que par exemple 2h à 80°C, 2h à 100°C, 2h à 110°C, 2h à 120°C, 2h à 130°C et 2h à 150°C.

Plus particulièrement le procédé selon l'invention peut comprendre entre les étapes b) et c) une étape b1) de recuit à une température supérieure à 200°C mais inférieure à la température de dégradation de la résine, par exemple à 220°C. Cette étape permet d'améliorer les propriétés thermomécaniques de la résine.

De façon avantageuse, l'enthalpie de polymérisation de l'étape b) est inférieure à 500 J / g.

Avantageusement, le taux de coke de la résine thermodurcie obtenue à l'étape b) est supérieur à 50%.

Dans un mode de réalisation avantageux, le matériau en résine thermodurcie, est un matériau formant la matrice d'un matériau composite du type comprenant une matrice dans laquelle se trouve un renfort.

Le renfort présent dans le matériau composite peut être de différents types. Ainsi, il peut notamment s'agir d'un renfort constitué de fibres telles que des fibres de verre, des fibres de quartz, des fibres de carbone, des fibres de graphite, des fibres de silice, des fibres métalliques comme des fibres d'acier ou des fibres d'aluminium, des fibres de bore, des fibres céramiques comme des fibres de carbure de silicium ou de carbure de bore, des fibres organiques de synthèse comme des fibres d'aramide, des fibres de polyéthylène, des fibres de polyester ou des fibres de poly(p-phénylène benzobisoxazole), plus connues sous le sigle PBO, des fibres organiques naturelles comme des fibres de chanvre, des fibres de lin ou des fibres de soie, ou encore de mélanges de telles fibres, auquel cas ce renfort peut se présenter, selon la nature des fibres qui le constituent, sous la forme de fils coupés, de fibres broyées, de mats à filaments continus, de mats à filaments coupés, de stratifils (ou « rovings » en langue anglaise), de tissus, de tricots, de feutres, ou encore sous la forme de complexes réalisés par association de différents types de matériaux plans.

II peut également s'agir d'un renfort constitué de particules telles que des particules de liège ou des charges réfractaires du type tungstène, oxyde de magnésium, oxyde de calcium, alumine, silice, dioxyde de zirconium, dioxyde de titane, oxyde de béryllium.

Par ailleurs, la fabrication du matériau composite, et donc l'ajout de renfort dans la résine, peut être réalisée par toutes les techniques connues de l'homme du métier des matériaux composites comme, par exemple, par imprégnation, par moulage par injection simultanée, par moulage par drapage autoclavé, par moulage sous vide, par moulage par injection basse pression de résine (ou RTM pour « Resin Transfert Molding »), par moulage à la presse à froid "voie humide" basse pression, par moulage par injection de compound (ou BMC pour « Bulk Molding Compound »), par moulage par compression de mats préimprégnés (ou SMC pour « Sheet Molding Compound »), par enroulement filamentaire, par centrifugation ou encore par pultrusion, l'imprégnation étant préférée dans le cas où le renfort est constitué de fibres.

De préférence, le matériau composite est un matériau composite ablatif et, plus spécifiquement, un matériau composite ablatif de protection thermique, notamment pour l'aérospatiale.

La présente invention sera mieux comprise à la lecture de la description des exemples qui suivent qui sont donnés à titre indicatifs.

Le dispositif utilisé pour les exemples comprend une colonne de réaction en verre d'une longueur de 64 cm et de diamètre de 3cm remplie de billes de verre sphériques de diamètre 4mm (±0,3mm), afin d'assurer l'échange thermique entre le réactif et le four, un four tubulaire électrique vertical, un récipient d'accueil de volume suffisant refroidit par azote liquide. Le dispositif comprend en outre une ampoule de coulée par laquelle introduire le réactif, dans le cas où la thermolyse est mise en œuvre en présence d'un solvant et une connexion à une pompe à membrane, permettant d'atteindre le vide nécessaire lorsque ce dernier est compris entre 1000 et 6000 Pa. Le dispositif comprend un tube horizontal d'introduction entouré de bandes chauffantes lorsque le réactif est évaporé/sublimé avant la thermolyse. Une pompe à palette est utilisée à la place de la pompe à membrane pour obtenir un vide de 5,5 Pa.

### Exemple 1 : Conversion du résorcinol propargylé et préparation de la résine selon l'invention

### Synthèse du résorcinol propargylé

10g (0,091 mol) de résorcinol (Alfa Aesar) est solubilisé dans 50 mL de diméthylsulfoxyde (DMSO), 50g (0,363 mol) de carbonate de potassium (K₂CO₃) est broyé puis ajouté sous agitation magnétique et le milieu est chauffé à 70°C (ext). 14,45 mL (2,2 eq.) de chlorure de propargyle (ABCR) est ajouté au goutte à goutte. La réaction est contrôlée par CCM avec un éluant 7:3 éther de pétrole : diéthyl éther (volume). Après filtration et dilution dans 100 mL d'acétate d'éthyle le milieu est extrait avec 3×100 mL de saumure. La phase organique est séchée sur MgSO₄, filtrée et concentrée sous pression réduite. Le composé est purifié par distillation sous vide (T°C = 120°C, 4,5 Pa). Le rendement est de 77,4%.

### Conversion du résorcinol propargylé

0,5 grammes (0,0027 mol) de résorcinol propargylé obtenu précédemment est dissout dans 20 mL de toluène et introduit dans l'ampoule de coulée en tête de colonne. Le réactif est introduit dans la colonne avec un débit moyen de 0,5 mL/minutes dans une colonne préchauffée entre 410°C et 430°C sous un vide de 6000 Pa. Après élution de la totalité du réactif et refroidissement de la colonne, le vide est coupé et le produit récupéré. Le toluène est évaporé sous pression réduite à l'évaporateur rotatif puis le produit séché à l'aide d'une pompe à palette. Le rendement brut obtenu est de 85%. La proportion de fonctions propargyle résiduelles est estimée par RMN ¹H inférieure à 1%. Le produit brut est composé de 13% molaire de C et 79.2% molaire de D. La proportion molaire de composé de type 2-indanone est de 7,8%. La proportion molaire de fonctions propargyle résiduelles inférieure à 11% est conforme au cahier des charges fixé.

### Polymérisation du chromène issu du résorcinol propargylé

La polymérisation des mélanges propargyle-chromène se fait par montée progressive en température. Dans le cas d'un mélange résorcinol propargyle-chromène avec une proportion en fonctions éthers de propargyle résiduelles inférieure à 11% tel qu'obtenu précédemment, le traitement thermique appliqué est le suivant : 2h à 80°C, 2h à 100°C, 2h à 110°C, 2h à 120°C, 2h à 130°C et 2h à 150°C.

Un recuit à 220°C peut être effectué pour augmenter les propriétés thermo-mécaniques.

Le taux de coke avant ou après recuit est de 64,3% et l'enthalpie de réaction est de 280 J/g.

### Exemple 2 : Conversion du résorcinol propargylé

### Conversion du résorcinol propargylé

10 grammes (0,0537 mol) de résorcinol propargylé obtenu comme dans l'exemple 1 sont dissouts dans 350 mL de toluène et introduits dans l'ampoule de coulée en tête de colonne. Le réactif est introduit avec un débit moyen de 1,5 mL/minutes dans une colonne préchauffée entre 420°C et 430°C sous un vide de 6000 Pa. Après élution de la totalité du réactif et refroidissement de la colonne, le vide est coupé et le produit récupéré. Le toluène est évaporé sous pression réduite à l'évaporateur rotatif puis le produit séché à l'aide d'une pompe à palette. Le rendement brut est de 83,4%. La proportion de fonctions propargyles résiduelles est estimée par RMN ¹H à 2,2%. Le brut est composé de 4,4% molaire de C et 71,3% molaire de D. La proportion molaire de composé de type 2-indanone est de 23,1%. La proportion molaire de fonctions propargyle résiduelles inférieure à 11% est conforme au cahier des charges fixé.

### Exemple 3 : Conversion de l'eugénol propargylé et préparation de la résine selon l'invention

### Synthèse de l'eugénol propargylé

L'eugénol (Sigma Aldrich) (200g), le K₂CO₃ (211g) et le diméthylformamide (DMF) (2000mL) sont introduits dans un ballon de 6L et sont chauffés à 75°C sous agitation mécanique. Le chlorure de propargyle (ABCR) à 70% dans le toluène (158,5 mL) est ajouté goutte à goutte à l'aide d'une ampoule de coulée et le milieu réactionnel est chauffé et agité à 75°C pendant la nuit. La réaction est contrôlée par CCM avec un éluant éther de pétrole/éther diéthylique 7:3 (volume). Après réaction, le milieu réactionnel est filtré puis dilué et rincé à l'acétate d'éthyle. La phase organique est rincée à l'eau jusqu'à décoloration de la phase aqueuse (4 fois). La phase organique est séchée sur MgSO₄ et concentrée sous vide. Le rendement du brut est de 93%. Le composé est purifié par distillation sous vide (p=4,5 Pa et T°C = 60°C). Le rendement du composé distillé est de 90%.

### Conversion de l'eugénol propargylé

0,5 gramme (0,0054 mol) d'eugénol propargylé obtenu précédemment est dissout dans 20 mL de toluène et introduit dans l'ampoule de coulée en tête de colonne. Le réactif est introduit dans la colonne avec un débit moyen de 0,5 mL/minutes dans une colonne préchauffée entre 410°C et 420°C sous un vide de 6000 Pa. Après élution de la totalité du réactif et refroidissement de la colonne, le vide est coupé et le produit récupéré. Le toluène est évaporé sous pression réduite à l'évaporateur rotatif puis le produit séché à l'aide d'une pompe à palette. Le rendement brut est de 65%. La proportion de fonctions propargyle résiduelles est estimée par RMN ¹H à 0%. La proportion molaire de composé chromène est estimée par RMN ¹H à 54%. Le composé phénolique parent du composé éther de propargyle, l'eugénol, est formé à hauteur de 46% molaire.

### Polymérisation du chromène issu de l'eugénol propargylé

Le procédé mis en œuvre est identique à celui décrit dans l'exemple 1 pour le résorcinol propargylé.

### Exemple 4 : Conversion de l'isoeugénol propargylé et préparation de la résine selon l'invention

### Synthèse de l'isoeugénol propargylé

20g (0,130 mol) d'isoeugénol (Sigma Aldrich) est solubilisé dans 100 mL (5eqm) de DMF. 33,67g (2eq.) de carbonate de potassium finement broyé (K₂CO₃) est ajouté sous agitation magnétique. 20,35 mL (1,5eq.) de bromure de propargyle (Alfa Aesar) (80%m dans le toluène) est ajouté à l'aide de l'ampoule de coulée. L'agitation magnétique est maintenue pendant 12h. La complétion de la réaction est contrôlée par CCM avec un éluant éther de pétrole : Acétate d'éthyle 70:30 (volume). Après filtration du K₂CO₃ et lavage à l'acétate d'éthyle, 100 mL d'acétate d'éthyle est ajouté dans le milieu pour l'extraction. La phase organique est lavée 3 fois à l'eau distillée (3×100 mL) et 1 fois à la saumure (1×100 mL). La phase organique est séchée sur MgSO₄, filtrée et concentrée sous pression réduite. Le rendement est de 91%.

Le composé est purifié par distillation sous vide au four à boule (p= 15 Pa et T°C chauffage = 140°C). Le composé est récupéré sous forme de cristaux blancs. Le rendement global après purification est de 73%.

### Conversion de l'isoeugénol propargylé

0,5 gramme (0,0054 mol) d'isoeugénol propargylé obtenu précédemment est dissout dans 20 mL de toluène et introduit dans l'ampoule de coulée en tête de colonne. Le réactif est introduit dans la colonne avec un débit moyen de 0,5 mL/minutes dans une colonne préchauffée entre 410°C et 420°C sous un vide de 6000 Pa. Après élution de la totalité du réactif et refroidissement de la colonne, le vide est coupé et le produit récupéré. Le toluène est évaporé sous pression réduite à l'évaporateur rotatif puis le produit séché à l'aide d'une pompe à palette. Le rendement brut est de 65%. La proportion molaire de fonctions propargyle résiduelles est estimée par RMN ¹H à 0%. La proportion molaire de composé chromène est estimée par RMN ¹H à 43%. Le composé phénolique parent du composé éther de propargyle, l'isoeugénol, est formé à hauteur de 57% molaire du mélange au cours de la réaction.

### Polymérisation du chromène issu de l'isoeugénol propargylé

Le procédé mis en œuvre est identique à celui décrit dans l'exemple 1.

### Exemple 5 : Conversion du résorcinol propargylé en absence de solvant et avec sublimation/évaporation du réactif et préparation de la résine selon l'invention

### Conversion du résorcinol propargylé

4 grammes (0,021 mol) de résorcinol propargylé obtenu comme dans l'exemple 1 sont dispersés dans 50 grammes de billes en verre de diamètre 4 mm et placés dans un tube horizontal en tête de colonne. La colonne est préchauffée entre 410 et 420°C. Le dispositif de thermolyse est placé sous un vide de 5,5 Pa. Le tube horizontal contenant le réactif est chauffé progressivement à une température de 130°C, permettant l'évaporation/la sublimation progressive du réactif et son passage dans la colonne. Le produit est récupéré en sortie dans un piège refroidit à l'azote liquide. Après élution de la totalité du réactif et refroidissement de la colonne, le vide est coupé et le produit récupéré. Le rendement brut est de 90%. La proportion de fonctions propargyle résiduelles est estimée par RMN ¹H à 0%. Le brut est composé de 14% molaire de C et 79% molaire de D. La proportion molaire de composé de type 2-indanone est de 7%. La proportion molaire de fonctions propargyle résiduelles inférieure à 11% est conforme au cahier des charges fixé.

### Polymérisation du chromène issu du résorcinol propargylé

Le procédé mis en œuvre est identique à celui décrit dans l'exemple 1.

### Exemple 6 : de conversion du résorcinol propargylé en absence de solvant et préparation de la résine selon l'invention

8 grammes (0,043 mol) de résorcinol propargylé obtenu comme dans l'exemple 1 sont introduits purs dans l'ampoule de coulée en tête de colonne. L'ampoule de coulée est chauffée à 40°C pour modifier l'état du réactif de solide à liquide. Le réactif est introduit dans la colonne avec un débit moyen de 0,5 mL/minutes dans une colonne préchauffée entre 410°C et 420°C sous un vide de 6000 Pa. Après élution de la totalité du réactif et refroidissement de la colonne, le vide est coupé et le produit récupéré. Le rendement brut est de 7%. La proportion de fonctions propargyle résiduelles est estimée par RMN ¹H à 0%. Le brut est composé de 3,3% molaire de C et 79,7% molaire de D. La proportion molaire de composé de type 2-indanone est de 17%.

### Polymérisation du chromène issu du résorcinol propargylé

Le procédé mis en œuvre est identique à celui décrit dans l'exemple 1.

### Exemple Comparatif 1 : tentative de conversion du résorcinol propargylé

1 gramme (0,0054 mol) de résorcinol propargylé obtenu comme dans l'exemple 1 est dissout dans 40 mL de toluène et introduit dans l'ampoule de coulée en tête de colonne. Le réactif est introduit dans la colonne avec un débit moyen de 0,5 mL/minutes dans une colonne préchauffée entre 250°C et 270°C sous un vide de 1000 Pa. Après élution de la totalité du réactif et refroidissement de la colonne, le vide est coupé et le produit récupéré. Le toluène est évaporé sous pression réduite à l'évaporateur rotatif puis le produit séché à l'aide d'une pompe à palette. Le rendement brut est de 92%. La proportion de fonctions propargyle résiduelles est estimée par RMN ¹H à 100%. La proportion de fonctions propargyle résiduelles supérieure à 11% est non conforme au cahier des charges fixé. La température est donc un paramètre important pour obtenir la conversion du résorcinol propargylé en chromène selon le cahier des charges fixé.

## Revendications

1. Procédé de fabrication de chromènes destinés à la préparation de résines thermodurcissables comprenant l'étape de transformation d'un éther de propargyle aromatique de formule générale (I) suivante 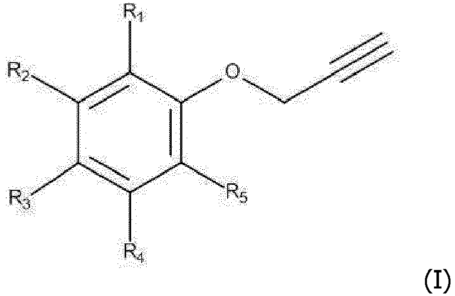 dans laquelle :
**R₁ et R₅** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₁ et R₅** représente un atome d'hydrogène ;
**R₂ et R₄** représentent indépendamment l'un de l'autre un atome d'hydrogène, un groupe alcène en C₂-C₆, alcyne en C₂-C₆ tel qu'un propargyle, un groupe O-alkyle en C₁-C₆ O-alcène en C₂-C₆ ou O-alcyne en C₂-C₆ tel qu'un O-propargyle ;
**et R₃** représente un atome d'hydrogène, un groupe O-alkyle en C₁-C₆ ou un groupe alcène en C₂-C₆, le groupe alcène étant éventuellement substitué par un groupe de formule générale (II) suivante 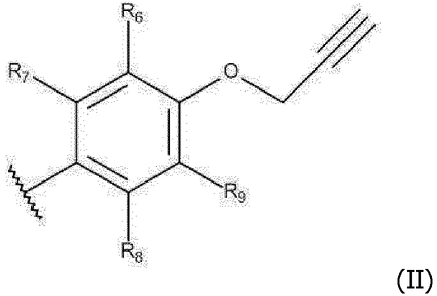 dans laquelle :
**R₆ et R₉** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₆ et R₉** représente un atome d'hydrogène ;
et **R₇ et R₈** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O- propargyle ;
à la condition que l'un au moins des **R₁, R₂, R₃, R₄ et R₅** ne représente pas un atome d'hydrogène ou un groupe O-alkyle en C₁-C₆ ;
et ses isomères cis/trans et ses isomères optiques et leurs mélanges racémiques
en un chromène par thermolyse éclair sous vide , à une température comprise entre 300 et 600°C, avantageusement entre 400 et 450°C, sous une pression comprise entre 4 et 10000 Pa, avantageusement entre 5 et 7000 Pa.

2. Procédé selon la revendication 1, **caractérisé en ce que** l'éther de propargyle aromatique de formule générale (I) est choisi dans le groupe constitué par le résorcinol propargylé, l'eugénol propargylé, l'eugénol couplé propargylé, l'isoeugénol couplé propargylé, l'isoeugénol propargylé et leurs mélanges et leurs isomères cis/trans et leurs isomères optiques et leurs mélanges racémiques, avantageusement il s'agit du résorcinol propargylé.

3. Procédé selon la revendication 2, **caractérisé en ce que** le pourcentage molaire de fonctions propargyles résiduelles du chromène est inférieur à 11% lorsque l'éther de propargyle aromatique de formule générale (I) est le résorcinol propargylé, le pourcentage molaire de fonctions propargyles résiduelles du chromène est inférieur à 39% lorsque l'éther de propargyle aromatique de formule générale (I) est l'eugénol couplé propargylé et le pourcentage molaire de fonctions propargyles résiduelles du chromène est inférieur à 35% lorsque l'éther de propargyle aromatique de formule générale (I) est l'isoeugénol couplé propargylé.

4. Procédé selon l'une quelconque des revendications 1 à 3, **caractérisé en ce que** l'éther de propargyle aromatique de formule générale (I) est au préalable solubilisé dans un solvant, avantageusement du toluène, avant l'étape de thermolyse éclair.

5. Procédé selon l'une quelconque des revendications 1 à 3, **caractérisé en ce que** l'éther de propargyle aromatique de formule générale (I) est au préalable évaporé/sublimé, avant l'étape de thermolyse éclair.

6. Procédé selon l'une quelconque des revendications 1 à 5, **caractérisé en ce qu'**il s'agit d'un procédé continu.

7. Procédé selon l'une quelconque des revendications 2 à 6, **caractérisé en ce que** l'éther de propargyle aromatique de formule générale (I) est le résorcinol propargylé et **en ce que** le chromène obtenu a la formule C et / ou D suivante, avantageusement est un mélange des formules C et D

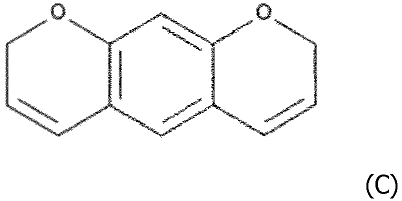

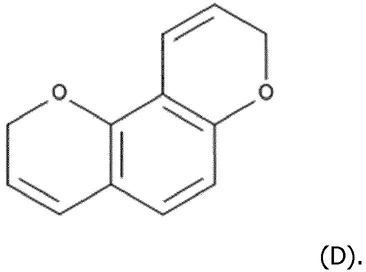

8. Procédé de préparation d'un matériau en résine thermodurcie comprenant les étapes successives suivantes :
- a) mise en œuvre du procédé selon l'une quelconque des revendications 1 à 6 ;
- b) polymérisation du produit de la réaction obtenu à l'étape a) de façon à obtenir le matériau en résine thermodurcie ;
- c) récupération du matériau en résine thermodurcie obtenue à l'étape b).

9. Procédé selon la revendication 8, **caractérisé en ce que** l'enthalpie de polymérisation de l'étape b) est inférieure à 500 J / g.

10. Procédé selon l'une quelconque des revendications 8 ou 9, **caractérisé en ce que** le taux de coke de la résine thermodurcie obtenue à l'étape c) est supérieur à 50% en masse.

## Patentansprüche

1. Verfahren zur Herstellung von Chromenen, die für die Herstellung von durch Wärme aushärtbare Harze bestimmt sind, umfassend den Schritt der Umwandlung eines aromatischen Propargylethers der nachstehenden allgemeinen Formel (I) 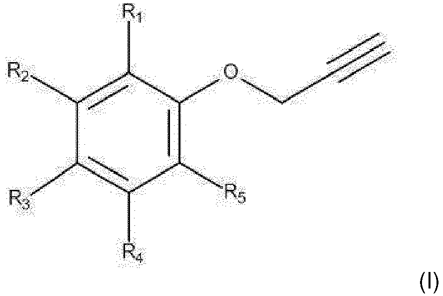 bei der:
R₁ und R₅ unabhängig voneinander ein Wasserstoffatom, eine C₂-C₆-Alken-, C₂-C₆-Alkin-, C₁-C₆ O-Alkyl-, C₂-C₆ O-Alken- oder C₂-C₆ O-Alkingruppe darstellen, mit der Maßgabe, dass wenigstens einer von R₁ und R₅ ein Wasserstoffatom darstellt,
R₂ und R₄ unabhängig voneinander ein Wasserstoffatom, eine C₂-C₆-Alken-, C₂-C₆-Alkingruppe, wie ein Propargyl, eine C₁-C₆ O-Alkyl-, C₂-C₆ O-Alken- oder C₂-C₆ O-Alkingruppe, wie ein O-Propargyl darstellen,
und R₃ ein Wasserstoffatom, eine C₁-C₆ O-Alkylgruppe oder eine C₂-C₆-Alkengruppe darstellt, wobei die Alkengruppe eventuell durch eine Gruppe der nachstehenden
allgemeinen Formel (II) substituiert ist 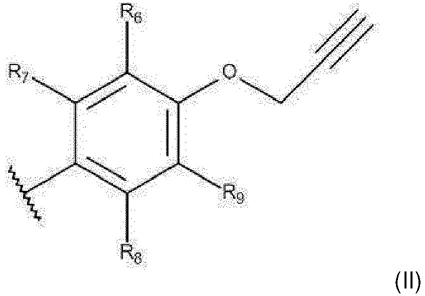 bei der:
R₆ und R₉ unabhängig voneinander ein Wasserstoffatom, eine C₂-C₆-Alken-, C₂-C₆-Alkin-, C₁-C₆ O-Alkyl-, C₂-C₆ O-Alken oder C₂-C₆ O-Alkingruppe darstellen, mit der Maßgabe, dass wenigstens einer von R₆ und R₉ ein Wasserstoffatom darstellt,
und R₇ et R₈ unabhängig voneinander ein Wasserstoffatom, eine C₂-C₆-Alken-, C₂-C₆-Alkin-, wie ein Propargyl, eine C₁-C₆ O-Alkyl-, C₂-C₆ O-Alken- oder C₂-C₆ O-Alkingruppe, wie ein O-Propargyl darstellen,
mit der Maßgabe, dass wenigstens einer von R₁, R₂, R₃, R₄ und R₅ kein Wasserstoffatom oder keine C₁-C₆ O-Alkylgruppe darstellt,
und seine cis/trans-Isomere und seine optischen Isomere und ihre racemischen Mischungen
in ein Chromen durch Flash-Vakuum-Thermolyse, bei einer Temperatur im Bereich zwischen 300 und 600 °C, vorteilhafterweise zwischen 400 und 450 °C, unter einem Druck im Bereich zwischen 4 und 10.000 Pa, vorteilhafterweise zwischen 5 und 7.000 Pa.

2. Verfahren nach Anspruch 1, **dadurch gekennzeichnet, dass** der aromatische Propargylether der allgemeinen Formel (I) ausgewählt ist aus der Gruppe bestehend aus Propargylresorcin, Propargyleugenol, gekoppeltes Propargyleugenol, gekoppeltes Propargylisoeugenol, Propargylisoeugenol und deren Mischungen und deren cis /trans-Isomeren und ihren optischen Isomeren und ihren racemischen Mischungen, vorteilhafterweise handelt es sich um Propargylresorcin.

3. Verfahren nach Anspruch 2, **dadurch gekennzeichnet, dass** der prozentuale Molanteil an restlichen Propargylfunktionen des Chromens kleiner als 11 % ist, wenn der aromatische Propargylether der allgemeinen Formel (I) Propargylresorcin ist, der prozentuale Molanteil an restlichen Propargylfunktionen des Chromens kleiner als 39 % ist, wenn der aromatische Propargylether der allgemeinen Formel (I) gekoppeltes Propargyleugenol ist, und der prozentuale Molanteil an restlichen Propargylfunktionen des Chromens kleiner als 35 % ist, wenn der aromatische Propargylether der allgemeinen Formel (I) gekoppeltes Propargylisoeugenol ist.

4. Verfahren nach einem der Ansprüche 1 bis 3, **dadurch gekennzeichnet, dass** der aromatische Propargylether der allgemeinen Formel (I) vor dem Schritt der Flash-Thermolyse in einem Lösungsmittel, vorteilhafterweise Toluol, zuvor gelöst wird.

5. Verfahren nach einem der Ansprüche 1 bis 3, **dadurch gekennzeichnet, dass** der aromatische Propargylether der allgemeinen Formel (I) vor dem Schritt der Flash-Thermolyse zuvor verdampft/sublimiert wird.

6. Verfahren nach einem der Ansprüche 1 bis 5, **dadurch gekennzeichnet, dass** es sich um ein kontinuierliches Verfahren handelt.

7. Verfahren nach einem der Ansprüche 2 bis 6, **dadurch gekennzeichnet, dass** der aromatische Propargylether der allgemeinen Formel (I) Propargylresorcin ist und dass das erhaltene Chromen die folgende Formel C und/oder D aufweist, vorteilhafterweise eine Mischung aus den Formeln C und D ist

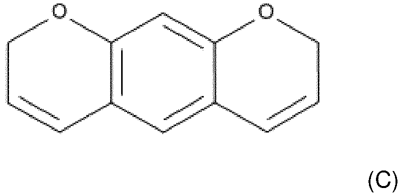

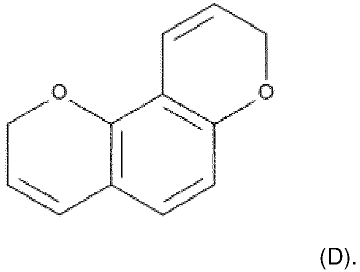

8. Verfahren zur Herstellung eines Materials aus durch Wärme ausgehärtetem Harz, umfassend die folgenden aufeinanderfolgenden Schritte:
- a) Durchführung des Verfahrens nach einem der Ansprüche 1 bis 6,
- b) Polymerisation des in Schritt a) erhaltenen Reaktionsproduktes, um das Material aus durch Wärme ausgehärtetem Harz zu erhalten,
- c) Gewinnung des Materials aus durch Wärme ausgehärtetem Harz, das in Schritt b) erhalten wurde.

9. Verfahren nach Anspruch 8, **dadurch gekennzeichnet, dass** die Polymerisationsenthalpie von Schritt b) weniger als 500 J/g beträgt.

10. Verfahren nach einem der Ansprüche 8 oder 9, **dadurch gekennzeichnet, dass** der Koksgehalt des in Schritt c) erhaltenen durch Wärme ausgehärteten Harzes größer als 50 Massen-% ist.

## Claims

1. Process for manufacturing chromenes which are intended for the preparation of thermosetting resins, comprising the step of transforming an aromatic propargyl ether of general formula (I) below 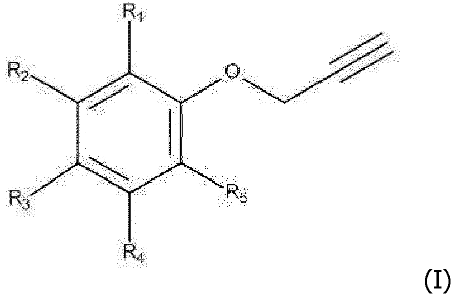 in which:
**R₁ and R₅** represent, independently of each other, a hydrogen atom, a C₂-C₆ alkene, C₂-C₆ alkyne, O-(C₁-C₆)alkyl, O-(C₂-C₆)alkene or O-(C₂-C₆)alkyne group, on condition that at least one from among **R₁ and R₅** represents a hydrogen atom;
**R₂ and R₄** represent, independently of each other, a hydrogen atom, a C₂-C₆ alkene, C₂-C₆ alkyne such as propargyl, O-(C₁-C₆)alkyl, O-(C₂-C₆)alkene or O-(C₂-C₆)alkyne group such as an O-propargyl;
and **R₃** represents a hydrogen atom, a O-(C₁-C₆)alkyl or a C₂-C₆ alkene group, the alkene group being optionally substituted with a group of general formula (II) below 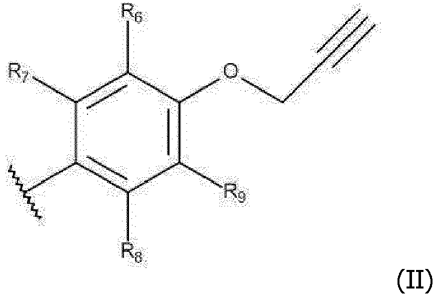 in which:
**R₆ and R₉** represent, independently of each other, a hydrogen atom, a C₂-C₆ alkene, C₂-C₆ alkyne, O-(C₁-C₆)alkyl, O-(C₂-C₆)alkene or O-(C₂-C₆)alkyne group, on condition that at least one from among **R₆ and R₉** represents a hydrogen atom;
and **R₇ and R₈** represent, independently of each other, a hydrogen atom, a C₂-C₆ alkene, C₂-C₆ alkyne such as propargyl, O-(C₁-C₆)alkyl, O-(C₂-C₆)alkene or O-(C₂-C₆)alkyne group such as an O-propargyl;
on condition that at least one from among **R₁, R₂, R₃, R₄ and R₅** does not represent a hydrogen atom or a O-(C₁-C₆)alkyl group;
and the cis/trans isomers thereof and the optical isomers thereof and the racemic mixtures thereof
into a chromene by flash vacuum thermolysis, at a temperature of between 300 and 600°C, advantageously between 400 and 450°C, at a pressure of between 4 and 10 000 Pa, advantageously between 5 and 7000 Pa.

2. Process according to Claim 1, **characterized in that** the aromatic propargyl ether of general formula (I) is chosen from the group consisting of propargylated resorcinol, propargylated eugenol, propargylated coupled eugenol, propargylated coupled isoeugenol, propargylated isoeugenol and mixtures thereof and the cis/trans isomers thereof and the optical isomers thereof and the racemic mixtures thereof; advantageously, it is propargylated resorcinol.

3. Process according to Claim 2, **characterized in that** the molar percentage of residual propargyl functions in the chromene is less than 11% when the aromatic propargyl ether of general formula (I) is propargylated resorcinol, the molar percentage of residual propargyl functions in the chromene is less than 39% when the aromatic propargyl ether of general formula (I) is propargylated coupled eugenol and the molar percentage of residual propargyl functions in the chromene is less than 35% when the aromatic propargyl ether of general formula (I) is propargylated coupled isoeugenol.

4. Process according to any one of Claims 1 to 3, **characterized in that** the aromatic propargyl ether of general formula (I) is dissolved beforehand in a solvent, advantageously toluene, before the flash thermolysis step.

5. Process according to any one of Claims 1 to 3, **characterized in that** the aromatic propargyl ether of general formula (I) is first evaporated/sublimed, before the flash thermolysis step.

6. Process according to any one of Claims 1 to 5, **characterized in that** it is a continuous process.

7. Process according to any one of Claims 2 to 6, **characterized in that** the aromatic propargyl ether of general formula (I) is propargylated resorcinol and **in that** the chromene obtained has the formula C and/or D below; advantageously, it is a mixture of formulae C and D

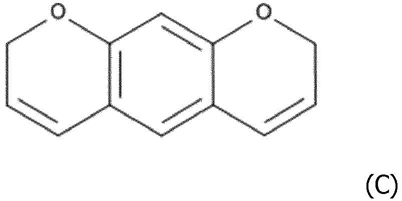

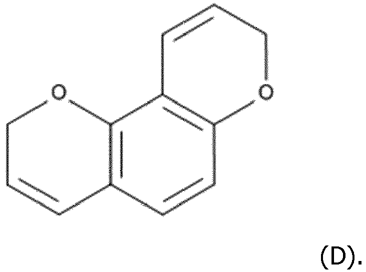

8. Process for preparing a material made of thermoset resin, comprising the following successive steps:
- a) implementation of the process according to any one of Claims 1 to 6;
- b) polymerization of the reaction product obtained in step a) so as to obtain the material made of thermoset resin;
- c) recovery of the material made of thermoset resin obtained in step b).

9. Process according to Claim 8, **characterized in that** the enthalpy of polymerization of step b) is less than 500 J/g.

10. Process according to either of Claims 8 and 9, **characterized in that** the coke content of the thermoset resin obtained in step c) is greater than 50% by mass.
